# EUROPEAN PATENT SPECIFICATION

(11) **EP 1 304 371 B1**
(45) Date of publication and mention of the grant of the patent: **03.01.2007**
(21) Application number: 01941154.5
(22) Date of filing: 21.06.2001
(51) Int. Cl.: C12N 5/10, C12N 15/12, A01K 67/027, A61K 31/711, A61K 38/00, A61K 45/00, A61K 48/00, A61P 35/00, C07K 14/47, G01N 33/15, G01N 33/50

(54) **CDS1 KNOCKOUT MICE AND CELL LINES ESTABLISHED THEREOF**
CDS1-KNOCKOUT-MÄUSEN UND DAVON ABGELEITETE ZELLLINIEN
SOURIS CDS1-KNOCKOUT ET DES LIGNEES CELLULAIRES ETABLIES A PARTIR DE CELLES-CI

(30) Priority: 21.06.2000 JP 2000191095; 12.12.2000 JP 2000378070
(43) Date of publication of application: 23.04.2003
(73) Proprietor: CHUGAI SEIYAKU KABUSHIKI KAISHA, Tokyo, 115-8543 (JP); Ikeda, Kyoji, Nagoya-shi, Aichi 458-0014 (JP)
(72) Inventor: IKEDA, Kyoji, Nagoya-shi, Aichi 458-0014 (JP); MOTOYAMA, Noboru, Nagoya-shi, Aichi 458-0015 (JP); TAKAI, Hiroyuki, Oobu-shi, Aichi 474-0022 (JP); WATANABE, Miho, Gotenba-shi, Shizuoka 412-0038 (JP)
(74) Representative: Vossius & Partner
(86) International application number: PCT/JP2001/005314
(87) International publication number: WO 2001/098465

(56) References cited:
- HIRAO A. ET AL.: 'DNA damage-induced activation of p53 by the checkpoint kinase Chk2' SCIENCE vol. 287, March 2000, pages 1824 - 1827, XP002945471
- DASIKA G.K. ET AL.: 'DNA damage-induced cell cycle checkpoints and DNA strand break repair in development and tumorigenesis' ONCOGENE vol. 18, 1999, pages 7883 - 7899, XP002945472
- TOMINAGA K. ET AL.: 'Role of human Cds1 (Chk2) kinase in DNA damage checkpoint and its regulation by p53' THE JOURNAL OF BIOLOGICAL CHEMISTRY vol. 274, no. 44, 1999, pages 31463 - 31467, XP002945473

## Description

### Technical Field

The present invention relates to Cds1 knockout and cells established from the mice.

### Background Art

Even among chemotherapeutic agents, the development of antitumor agents is socially very significant since cancer is one of the three major diseases. Until now, various kinds of antitumor agents have been developed and clinically used. However, they are not satisfactory from the view points of effectiveness and side effects, and the development of new antitumor agents that have more cancer cell selectivity, less side effects, and also superior effects is anticipated. So far, DNA-damaging antitumor agents represented by alkylating agents and platinum complex-type antitumor agents have been developed.

When DNA in a cell is damaged, the cell generally has a mechanism to raise its viability, namely cell cycle checkpoints, by which the cell cycle, and thus cell division, are halted to prevent the accumulation of damaged DNA (Hartwell, L. H. and Weinert, T. A. : Science, 246, 629-634, 1989). A cell that has damaged DNA normalizes its DNA by repairing it while cell division is halted at the cell cycle checkpoints before resuming proliferation. When cell division cannot be stopped at cell cycle checkpoints, cells that have damaged DNA continue to proliferate and are finally killed by apoptosis. Due to such mechanisms, only cells that do not have damaged DNA are thought to proliferate.

The mechanism of checking DNA damage through cell cycle checkpoints is the same in both cancer cells and normal cells. Thus , it is being revealed that cancer cells can proliferate even if the DNA in these cancer cells are damaged by antitumor agents or irradiation that induces DNA damage, since the DNA damaged by the antitumor agents or irradiation is repaired due to cell cycle. On the contrary, if only the cell cycle checkpoints can be inhibited, the division of cancer cells can be promoted without repairing the DNA damage induced by the antitumor agent or irradiation. As a result, it is thought apoptosis will be induced, which would selectively kill the cancer cells.

The G1/S checkpoint and the G2/M checkpoint are the presently known cell cycle checkpoints. It was revealed that p53, which is a product of a tumor suppressor gene, plays an important role in the G1/S checkpoint (Kuerbitz,S. et al.: Proc.Natl.Acad.Sci.USA, 89, 7491-7495, 1992; Hartwell, L.H. et al.: Science, 266, 1821-1825, 1994). Later, it was also revealed that cancer cells that lack p53 can somehow stop the cell cycle in response to DNA damage and can efficiently repair the damaged DNA (Deng.C. et al. : Cell, 82, 675, 1995; Waldman, T. et al.:Cancer Res., 55, 5187, 1995; Brown,J.P. et al. : Science, 277, 831, 1997; Powel,S.N. et al.: Cancer Res, 55, 1643, 1995). This suggested the presence of a checkpoint that is different from the G1/S checkpoint in which p53 is involved, namely the G2/M checkpoint. Cds1 (Chk2) has already been identified as a molecule that plays an important role in the G2/M checkpoint (Matsuoka,S. et al.: Science, 282, 1893, 1998)

p53-deficient or p53-mutated cells are clinically observed in lung cancer, colon cancer, pancreatic cancer, and so on. It may be possible to' specifically kill cancer cells by targeting Cds1 (Chk2) and inhibiting the p53-independent checkpoint, the G2/M checkpoint, in these cells. Recently, it was revealed that Cds1 (Chk2) is involved in not only the G2/M checkpoint, but also cell cycle arrest, repair, and apoptosis by stabilizing p53 (Hirao, A. et al.: Science, 287, 1824, 2000).

However, the action of Cds1 (Chk2) has not been revealed yet, and experiments are required to examine whether the inhibition of Cds1 (Chk2) can induce cancer cell-specific cell death and what kind of side effects can be expected. Although ES cells and thymocytes in which both Cds1 (Chk2) alleles are inactivated are known (Hirao,A. et al.: Science, 287, 1824, 2000), it was impossible to analyze the molecule at a whole individual level.

If genetically modified mice and ES cell lines, which have no enzymatic activity of Cds1 (Chk2), and cell lines established from the genetically modified mice are constructed by inactivating both alleles of Cds1 (Chk2) of the mice, such mice and cell lines can be utilized for the elucidation of the physiological function of Cds1 (Chk2) in variously differentiated cells at whole individual and cellular levels. As models that lack a cell cycle checkpoint, they can also be used to analyze the sites of action of anticancer drugs, to search for novel drugs that have an effect of inhibiting a checkpoint, and to determine the effect of novel drugs that can inhibit a checkpoint at an individual level. Thus, such mice and cell lines are useful for obtaining important information to develop novel anticancer drugs.

### Disclosure of the Invention

An objective of the present invention is to provide genetically modified mice, and cell lines established from the genetically modified mice, in which both of Cds1 (Chk2) alleles are inactivated and which are useful for developing novel anticancer drugs. The present invention further describes uses of genetically modified mice, ES cell lines, and cell lines established from the genetically modified mice.

As a result of exhaustive investigations conducted with the aim of solving the above-mentioned problems, the present inventors established ES cell lines in which one of the Cds1 (Chk2) alleles was inactivated using gene targeting. They also constructed chimeric mice using the cell lines, and constructed genetically modified mice in which both alleles of Cds1 (Chk2) were inactivated. By incubating the ES cell line in which one locus was inactivated in a selective medium with a high concentration of an antibiotic, ES cell lines in which the other locus was also inactivated were constructed, the present invention provides genetically modified mice in which an exogenous gene has been inserted into both of Cds1 (Chk2) alleles and cell lines that are established from the genetically modified mice.

The genetically modified mice of the present invention can be constructed as follows. First, DNA including the exons of Cds1 (Chk2) gene is isolated from a mouse. Then, a targeting vector is constructed by inserting an appropriate marker gene to this DNA fragment. This targeting vector is introduced into a mouse ES cell line by electroporation method and such, and a cell line in which homologous recombination has occurred is selected. As a marker gene to be inserted, a gene resistant to an antibiotic such as the neomycin-resistant gene is preferred. When a gene resistant to an antibiotic is inserted, a cell line in which homologous recombination has occurred can be easily selected merely by incubating the cells in a medium that contains the antibiotic. For a more effective selection, preferably, a gene such as the thymidine kinase gene is inserted into the targeting vector. By this procedure, cell lines in which non-homologous recombination has occurred can be excluded. A diphtheria toxin A fragment (DT-A) gene and such can also be inserted into the targeting vector. Cell lines in which non-homologous recombination has occurred can be excluded by this method.

A cell line in which one of the Cds1 (Chk2) alleles are inactivated can be selected.by the above-mentioned manipulation . A chimeric mouse can be constructed by injecting the obtained ES cell line into a mouse blastocyst. A mouse in which one Cds1 (Chk2) allele is inactivated can be obtained by crossing this chimeric mouse with a wild type mouse. Furthermore, by crossing the offspring produced with each other, a mouse in which both Cds1 (Chk2) alleles are inactivated can be obtained. An ES cell line in which both of Cds1 (Chk2) alleles are inactivated can be obtained by the method described in the present Examples, namely by incubating an ES cell line in which one locus is inactivated in a selective medium with a high concentration of an antibiotic. The above-mentioned ES cell line can also be constructed by selecting an ES cell line in which one locus is inactivated, introducing the targeting vector into this cell line again, and selecting a cell line in which homologous recombination has occurred. As a marker gene inserted into the targeting vector, it is preferred that a marker gene different from that used previously is used.

Conventional methods can be used for establishing cell lines from the genetically modified mice of the present invention. For example, the primary culture method for rodent embryonic cells can be used (Shin-seikagaku Jikken Kouza, vol. 18, pp 125-129, Tokyo Kagaku Doujin; and "Manuals for manipulating mouse embryos", pp 262-264, Kindai Shuppan).

The geneticallymodifiedmice, ES cells and cell lines established from the mice of the present invention can be utilized, for example, for highly sensitive detection of the effects and side effects of anticancer drugs targeting DNA and for analyzing the mechanisms of cell cycle arrest, apoptosis, ahd DNA repair.

For example, the genetically modified mouse and mouse ES cell lines of the present invention can be used for predicting the effects of an inhibitor of Cds1 (Chk2). It was revealed that an inhibitor of Cds1 (Chk2) did not have lethal effects since genetically modified mice obtained from the present invention grew normally and did not die, at least during fetal development. Furthermore, it is possible to predict effects of an inhibitor of Cds1 (Chk2) by a detailed examination of the genetically modified mice. Effects of an inhibitor of Cds1 (Chk2) in each tissue can be precisely examined by using cell lines established from tissues of the genetically modified mice.

Whether a disease is due to the malfunction of Cds1 (Chk2) or not can be revealed by observing the symptoms of the genetically modified mice of this invention and comparing them with the symptoms of diseases whose cause has not been known until now. For example, when a characteristic phenotype appearing in a genetically modified mouse, or cell line derived from the mouse, is observed, it is compared with various symptoms of a human disease. If more than a half of the symptoms of the human disease are observed in the genetically modified mice of this invention, it can be presumed that the disease may be due to the malfunction of Cds1 (Chk2).

The genetically modified mice, and cell lines derived from the genetically modified mice of this invention lack the signal transduction mechanism involved in the G2/M checkpoint and the mechanism of maintaining the cell cycle, since Cds1 is inactivated. Thus, the ability to maintain and modify DNA at the G2/M checkpoint is inhibited when DNA is damaged. In other words, the G1/S checkpoint is the only checkpoint where DNA can be repaired in the genetically modified mice and cell lines derived from the genetically modified mice of this invention. Thus, anticancer drugs involved in the G1/S checkpoint can be screened with a high-sensitivity and efficiency by using these mice and cells.

Cell lines and genetically modified mice of this invention can be utilized for analyzing mechanisms of cell cycle arrest and apoptosis by comparing the difference in the response to cell cycle arrest or apoptosis, which is induced by DNA damage by X-rays or by drugs causing DNA damage, between the cell lines or genetically modified mice of this invention and normal cells.

The cell lines and genetically modified mice of this invention can be used for examining whether or not a test protein can substitute for Cds1 (Chk2) and for identifying such a protein. Furthermore, they can be used for screening for DNA encoding a protein that can substitute for Cds1 (Chk2).

For example, the restoration of G2 arrest in a cell line of the present invention into by introducing a novel gene or protein under existence of a drug that inhibits the G1/S checkpoint can confirm that the gene or protein induced can be a substitute Cds1 (Chk2). Thus, a cell line of this invention can be used for identifying a novel gene or protein that can substitute for Cds1 (Chk2). When a DNA library is introduced into the cell line and the same experiment is conducted, a novel gene or protein that can substitute for Cds1 (Chk2) can be screened. The thus isolated novel gene or protein substituting for Cds1 (Chk2) can be utilized for treating a disease that is caused by the malfunction of Cds1 (Chk2).

ES cell lines and cell lines derived from the genetically modified mice of this invention can be used for screening for a compound that induces the G2/M checkpoint. Because these cells lack the G2/M checkpoint, the period of G2 arrest is short. Thus, a drug that induces the G2/M checkpoint can be identified as follows. First, a test compound is contacted with cells that are treated with X ray irradiation or drugs causing DNA damage. And then, whether the cell can maintain G2 arrest as long as normal cell do is examined and used as an index to screen a compound. To detect G2 arrest, DNA is stained with propidium iodide, and such, and detected by FACS.

The cell lines and genetically modified mice of this invention can be used for analyzing the mechanism of DNA repair. After a cell line or mouse of this invention is X-ray irradiated or a drug causing DNA damage is administered, the activity to repair the cleaved DNA is measured (Tanaka, H., et al.: Nature 404, 42-49 (2000), or Shin-seikagaku Jikken Kouza 2, 139-200). The same manipulation is conducted to a normal cell. If a decrease or increase of activity to repair cleaved DNA is observed compared to that of normal cell, it can be confirmed that Cds1 (Chk2) was involved in the in DNA repair activity. If the DNA repair activity increases, a novel protein possessing DNA repair activity can be identified by identifying mRNA produced specifically. If the activity decreases, a compound that can induce DNA repair activity or a gene encoding a novel protein that has DNA repair activity can be screened. For example, DNA repair activity is measured using a cell of the present invention that is X-irradiated or incubated with a drug causing DNA damage as a control. On the other hand, DNA repair activity of a cell that is cultured with a test sample or a cell into which a gene is introduced using a DNA library is measured. If the DNA repair activity of the above-mentioned cell is increased, it is revealed that the test compound induces DNA repair activity or that the gene introduced encodes a novel protein that has DNA repair activity.

Furthermore, if mice and cells that lack both genes can be obtained by mating the mice of the present invention, or the mice obtained from the mouse ES cell line, or cell line of this invention with other mice in which a gene involved in the cell cycle, apoptosis, and checkpoint has been knocked out, these mice and cells can be efficiently utilized for constructing an evaluation system for drugs and for identifying target molecules of drugs such as anticancer drugs.

The compounds that can be isolated by the screening methods of the present invention can be used as pharmaceutical agents for humans and non-human animals such as mice, rats, guinea pigs, rabbits, chickens, cats, dogs, sheep, pigs, cows, monkeys, baboons, or chimpanzees, by directly administering the isolated compound itself into the patient, or by formulating it according to pharmacologically accepted methods and then administered. For example, the compound(s) may be orally administered as tablets, which may be sugar-coated as necessary; as capsules; elixirs; and microcapsules, or parenterally administered as injections, in the form of an aseptic solution or suspension with water or other pharmaceutically acceptable liquid. For example, a pharmaceutical composition may be formulated into a unit dose form generally required for drug implementation by suitably mixing with a pharmacologically acceptable carrier or medium, specific examples of which include sterilized water or physiological saline, vegetable oil, an emulsifier, a suspension agent, surfactant, stabilizer, flavoring agent, excipient, vehicle, antiseptic, binder, and so on. The amount of active ingredient in these preparations is adjusted so as to obtain a suitable volume within a specified range.

Examples of additives that can be mixed into tablets and capsules include binders, such as gelatin, cornstarch, gum tragacanth, and gum arabic; excipients, such as crystalline cellulose; swelling agents, such as cornstarch, gelatin, and alginic acid; lubricants, such as magnesium stearate; sweeteners, such as sucrose, lactose, and saccharin; and flavoring agents, such as peppermint, *Gaultheria adenothrix* oil, and cherry flavoring. When the preparation unit is in the form of a capsule, liquid carriers, such as fats and oils, can be contained in addition to the above materials. Aseptic compositions for injection can be formulated according to ordinary preparation methods using vehicles, such as distilled water for injection

Examples of aqueous solutions suitable for injections include physiological saline, and isotonic liquids containing glucose or other adjuvants, such as D-sorbitol, D-mannose, D-mannitol, and sodium chloride. These may be used in combination with suitable solubilizers, examples of which include alcohols such as ethanol; polyalcohols including propylene glycol and polyethylene glycol; and nonionic surfactants, such as Polysorbate 80 (TM) and HCO-50.

Examples of oleaginous liquids include sesame oil and soybean oil, and these may be used in combination with solubilizers, such as benzyl benzoate and benzyl alcohol. In addition, buffers, such as phosphate buffer and sodium acetate buffer; analgesics, such as procaine hydrochloride; stabilizers, such as benzyl alcohol and phenol; and antioxidants may also be blended. The prepared injection liquid is normally filled into suitable ampules.

Administration to patients can be carried out by methods known to those skilled in the art, for example, by intra-arterial injection, intravenous injection or subcutaneous injection; or intranasal, transbronchial, intramuscular, percutaneous or oral administration. Although the dosage varies depending on the body weight and age of the patient, the administration method, and so on, an appropriate dosage can be suitably selected by a person skilled in the art. In addition, if the compound can be encoded by a DNA, gene therapy may also be carried out by incorporating the DNA into a vector for gene therapy. Although the dosage and administration method vary according to the body weight, age, and symptoms of the patient, they can be suitably selected by a person skilled in the art.

Although the dosage of the compound varies according to the symptoms, in the case of oral administration, the dosage for an adult (with a body weight of 60 kg) typically ranges from about 0.1 to 100 mg per day, preferably about 1.0 to 50 mg per day, and more preferably about 1.0 to 20 mg per day.

In the case of parenteral administration, although varying according to the administered subject, target organ, symptoms, and administration method, a single dosage in the form of an injection preparation, for example, for an adult (with a body weight of 60 kg) is normally about 0.01 to 30 mg per day, preferably about 0.1 to 20 mg per day, and more preferably about 0.1 to 10 mg per day; and the administration is preferably carried out by intravenous injection. When administering to other animals, doses converted to 60 kg body weight or per body surface area can be administered.

### Brief Description of the Drawings

Fig. 1 depicts photographs showing the change in the amount of p53 protein in Cds1-/-ES cells with time after irradiation. The analysis was done by western blotting. The induction of p53 expression was observed in Cds1-/-ES cells as well as in the wild type after 60 minutes. However, the amount of expression was decreased in Cds1-/-ES cells. In addition, the induction of p53 expression was inhibited by caffeine in both Cds1-/-ES cells and wild type.
Fig. 2 shows the cell cycle distribution of Cds1 (Chk2)+/- or -/-ES cells after irradiation. After ES cells were X-ray irradiated, DNA was labeled with bromodeoxyuridine (Br-dU) . Then, the cell cycle was analyzed by FACS. Any changes due to the addition of caffeine after the irradiation was also compared. There was no difference in cell cycle distribution between Chk2+/- and Chk2-/-ES cells when cells were not irradiated. No obvious difference in cell cycle distribution was observed after 6 hours even after irradiation or caffeine treatment.

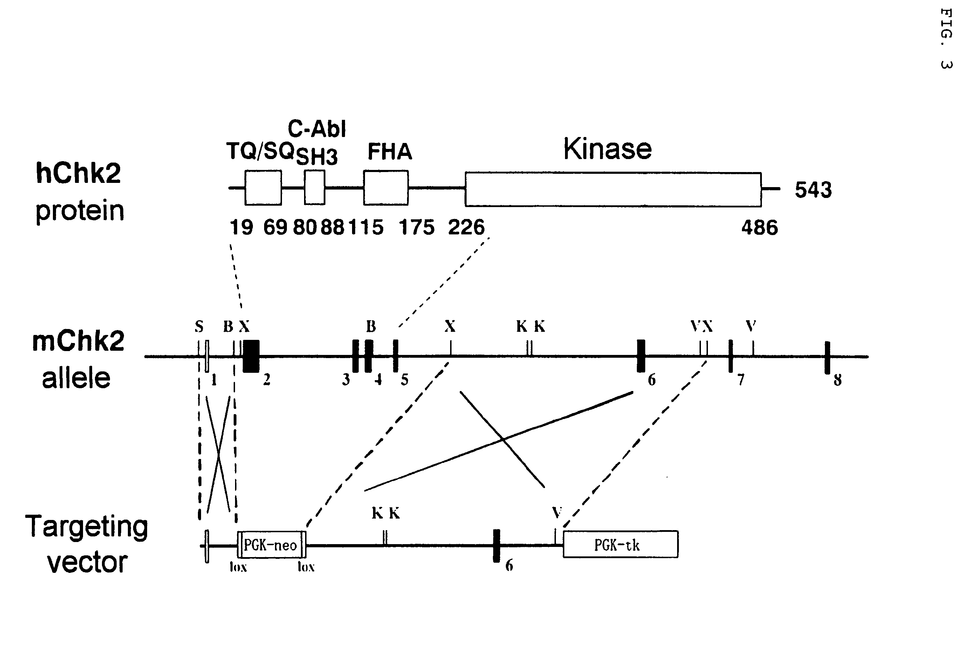

Fig. 3 shows the structure of Cds1 (Chk2) locus, and the structure of the homologous recombination vector which was used to introduce the mutated gene into the locus. Gene fragments containing exon 1 and exon 6 of the mouse Cds1 (Chk2) gene were respectively connected to the two ends of the expression cassette of neomycin-resistant gene so that the region from exon 2 to exon 5 was replaced by the expression cassette of neomycin-resistant gene by homologous recombination. Expression cassette of tk was connected to the downstream of the 3' end. The homologous recombination vector was constructed in this manner.

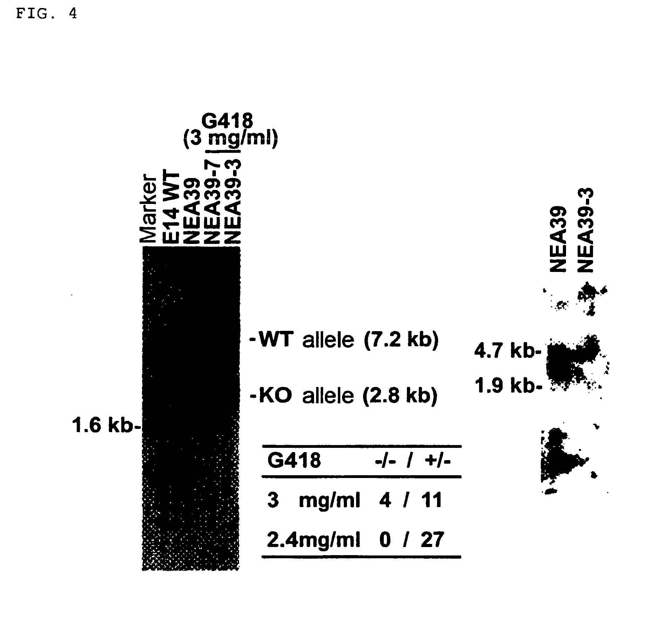

Fig. 4 depicts photographs showing the result of genotype analysis of ES cells in which one or both of the Cds1 (Chk2) alleles were inactivated. The results of Cds1 (Chk2) gene expression analysis are also shown. The normal Cds1 (Chk2) locus has a 7.2kb fragment, while the gene locus into which mutant Cds1 (Chk2) gene was inserted by homologous recombination has a 2.8kb fragment (left) . ANEA39-3 strain in which the mutant Cds1 (Chk2) gene was inserted into both alleles of Cds1 (Chk2) was obtained from NEA39 strain in which the mutant Cds1 (Chk2) gene had been inserted into one of the alleles by selective cultivation with 3 mg/ml of G418. While NEA39 strain showed the expression of Cds1 (Chk2) mRNA, the NEA39-3 strain showed no expression (right).

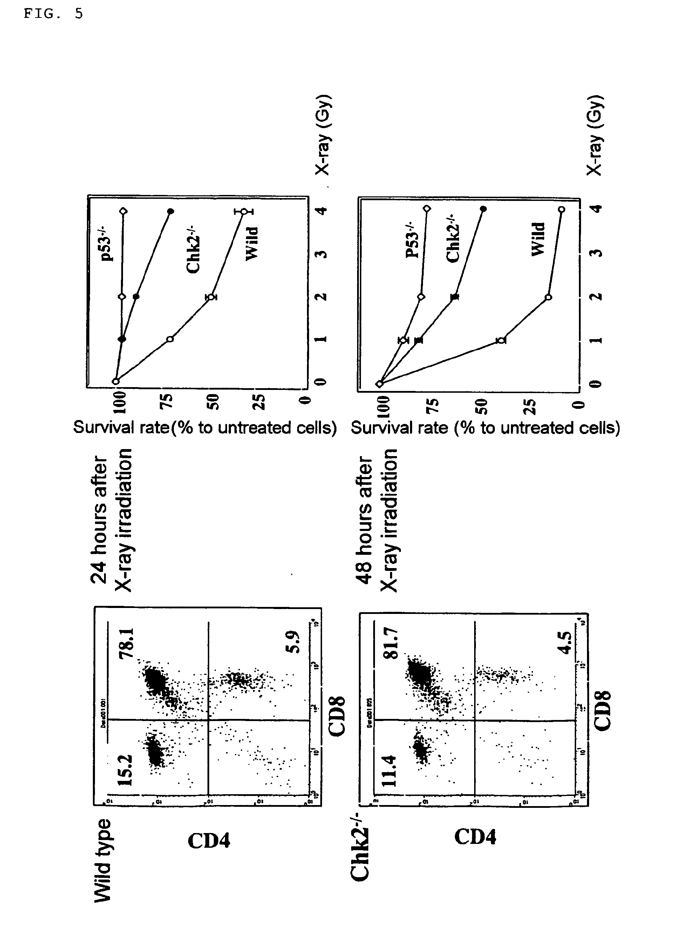

Fig. 5 shows the radiosensitivity of Chk2-/- and p53-/- thymocytes prepared using a chk2-/- mouse and p53-/- mouse and double-stained with antibodies against CD4 and CD8. The distribution was analyzed using a cell sorter (left) . CD4 or CD8 positive cells showed the same distribution as Chk2-/- or Chk2+/+ thymocytes. Survival rate of thymocytes 24 hours and 48 hours after the irradiation with 10Gy X-ray was measured (right). As a result, although irradiation-induced apoptosis of Chk2-/- thymocytes was decreased compared to that of the wild type, the resistance was less compared to that of p53-/ thymocytes.
Fig. 6 shows the X ray irradiation-induced change of the cell cycle of Chk2-/- and p53-/- mouse embryonic fibroblasts. Chk2-/- and p53-/- mouse embryonic fibroblasts were prepared and irradiated with 10Gy X-ray. Then, the distribution of the cell cycle at 0 hours and 12 hours after irradiation was analyzed using FACS. The Chk2-/- mouse embryonic fibroblasts showed the same cell cycle distribution as wild type before and after X-ray irradiation. On the other hand, p53-/ mouse embryonic fibroblasts showed a stronger suppression of G1 arrest after irradiation.
Fig. 7 shows the change of the survival rate of Cds1 (Chk2)-/-, Cds1 (Chk2) +/-, and wild type mice after irradiation. Cds1 (Chk2)-/mice have lower irradiation-induced death rates than Cds1 (Chk2)+/ or wild-type mice.
Fig. 8 depicts photographs showing an overview of mice 3 months after the irradiation. Depigmentation of the hair coat was observed in both Cds1 (Chk2)-/- and wild-type mice, however, the Cds1 (Chk2)-/ mouse had a wider area of depigmentation. A white line has been added to clarify the border.
Fig. 9 depicts graphs showing the result of histological tests after the irradiation. A decrease in the number of leukocytes and platelets was observed in all genotypes. An irradiation-induced change in the hematocrit value was not observed in any of the genotypes .
Fig. 10 depicts photographs showing apoptosis in the small intestine of newborns after the irradiation. A decrease in the number of cells showing apoptosis was observed in Cds1 (Chk2)-/- mouse.
Fig. 11 depicts photographs showing apoptosis in the thymus of newborns after the irradiation. A decrease in the number of apoptotic cells was observed in Cds1 (Chk2)-/- mouse.
Fig. 12 depicts photographs showing apoptosis in the granule cell layer in the cerebellum of newborns after the irradiation. A decrease in the number of apoptotic cells was observed in Cds1 (Chk2) -/-mouse.
Fig. 13 shows the amount of expression of p53 protein that was induced by irradiating thymocytes of Cds1 (Chk2)-/- and wild-type mice. It was revealed that Cds1 (Chk2)-/- mouse has a less amount of p53 protein than the wild-type mouse.
Fig. 14 shows the amount of expression of genes that are considered to be controlled by p53. The expression was induced by irradiating thymocytes of Cds1 (Chk2)-/- and wild-type mice. It was revealed that the Cds1 (Chk2)-/- mouse has a decreased expression of p21 and Noxa.
Fig. 15 shows populations of thymocytes after irradiation of Cds1-/- and wild-type mice. The analysis was done by FACS. It was shown that wild-type mice had a sensitivity to irradiation since a decrease in CD4 and CD8 double positive cells were observed in wild-type mice. On the other hand, since there are no decrease in CD4 and CD8 double positive cells in Cds1-/- mice, it was shown that Cds1-/- mice had decreased the sensitivity to irradiation, namely resistance, similar to the p53-/- mice.

### Best Mode for Carrying out the Invention

The present invention shall be described in detail below with reference to Examples , but is not be construed as being limited thereto.

### [Example 1] Construction of a vector to be used for the homologous recombination of Cds1 (Chk2) gene

In this Example, the mouse genomic sequence of Cds1 (Chk2) was cloned to construct a vector to be used for homologous recombination of Cds1 (Chk2) gene. Plaque hybridization was conducted using a liver cDNA library (Lamda FIXII, Stratagene) of mouse (129/SvJ) and open reading frame of human Cds1 cDNA as a probe. An approximately 20kb genomic DNA fragment containing exon 1 of Cds1 (Chk2) gene was isolated, and a restriction map was constructed. A tk expression cassette was connected to 3'-side downstream of a 6kb DNA fragment containing of exon6 of Cds1(Chk2) gene. The vector for homologous recombination was constructed so that it can replace the region from exon2 to 5 of Cds1 (Chk2) gene with an expression cassette of neomycin-resistant gene (Neo^{r})

### Reference [Example 2] Establishment of ES cell lines in which one of the Cds1 (Chk2) alleles is inactivated with mutant Cds1 (Chk2) gene insertion by homologous recombination

In this Example, homologous recombination vector was introduced to mouse ES cell E14 (Hooper,M. et al.: Nature, 326, 292-295, 1987) by electroporation method, and selectively cultured using G418 and ganciclovir. The obtained G418/ganciclovir-resistant colony was tested for A homologous recombinants by PCR and southern blotting. As a result, 5 clones having the mutant Cds1 (Chk2) gene inserted to one of the Cds1 (Chk2) alleles were obtained.

### Reference [Examples 3] Establishment of ES cell lines in which both Cds1 (Chk2) alleles are inactivated with mutant Cds1 (Chk2) gene insertion by homologous recombination'

In this example, ES cell lines in which one of the Cds1 (Chk2) alleles was inactivated with mutant Cds1 (Chk2) gene insertion by the homologous recombination vector was cultured in selective medium with a high concentration of G418. As a result, four clones in which both allele of Cds1 (Chk2) gene were inactivated, of the present invention, were obtained. In the obtained clones, the insertion of mutant gene was confirmed by southern blotting analysis, and the inactivation of both Cds1 alleles was confirmed by northern blotting analysis (Fig. 4).

### Reference [Example 4] Production of chimeric mice using ES cells in which one of the Cds1 (Chk2) alleles is inactivated

A chimeric mouse was obtained by injecting ES cell lines in which one of Cds1 (Chk2) alleles was inactivated into blastocysts derived from C57BL/6 mice.

### [Example 5] Production of mice in which both Cds1 (Chk2) alleles are inactivated

A mouse having cells in which one of Cds1 (Chk2) alleles is inactivated (Cds1 (Chk2)+/- mouse) was obtained by mating a C57BL/6 mouse with a chimeric mouse produced using ES cells in which one of the Cds1 (Chk2) alleles was inactivated. A mouse in which both Cds1 (Chk2) alleles are inactivated (Cds1 (Chk2)-/- mouse) was obtained by mating these Cds1 (Chk2)+/- mice with each other.

### [Example 6] Expression analysis of Cds1 (Chk2) gene in ES cell lines or in a mouse in which both Cds1 (Chk2) alleles were inactivated

The ES cell line or mouse (Cds1 (Chk2)-/- mouse) in which both Cds1 (Chk2) alleles are inactivated did not die. Thus, multiple lines of mouse and cell lines could be constructed. No expression of Cds1 (Chk2) mRNA was observed in any line.

### Reference [Example 7] Evaluation of the cell cycle arrest and the stabilization of p53 against X ray irradiation using the ES cell lines in which one or both Cds1 (Chk2) alleles are inactivated

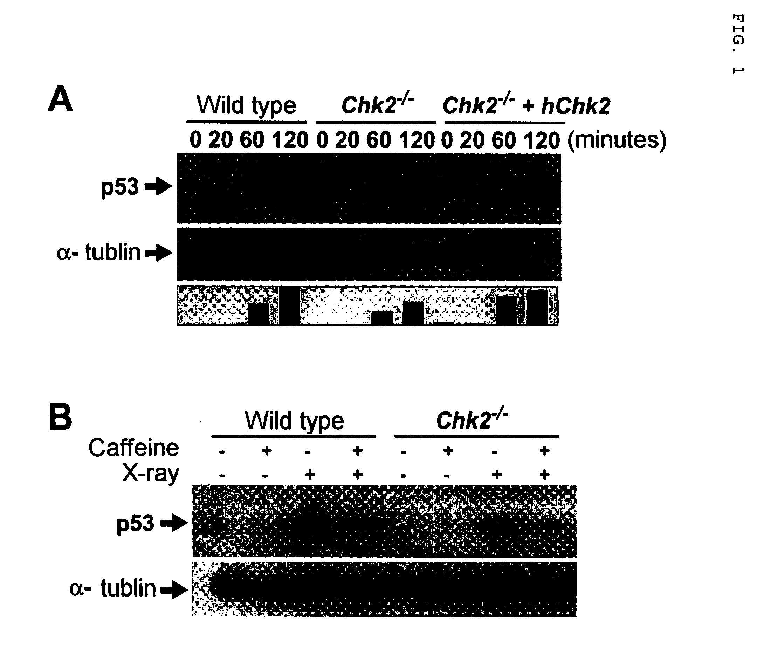

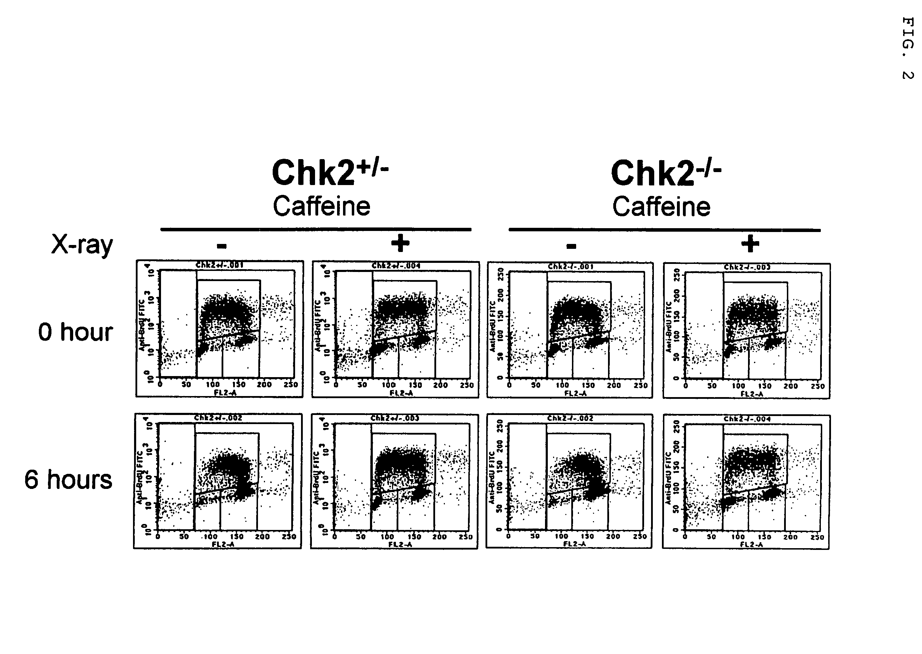

ES cell lines in which one or both Cds1 (Chk2) alleles were inactivated (Cds1 (Chk2)+/- and Cds1 (Chk2)-/-) were treated with trypsin and counted. Then, a constant number of ES cells were plated in 6-well plates in which feeder cells had been planted in advance. MEF (mouse embryonic fibroblast) was used as feeder cells. The cells were treated with X ray and cultured in an CO₂ incubator for 6 hours. Then, a cell extract was prepared, the proteins were separated by SDS-PAGE, and transferred to a PVDF membrane. p53 protein was quantified by conducting western blotting using an antibody against p53. As a result, p53 was stabilized and increased in Cds1 (Chk2)+/cells, while it was not stabilized in Cds1(Chk2)-/- cells as shown in Fig. 1. Furthermore, it was presumed that stabilization of p53 in Cds1-/- cells was dependent on the pathway same as that of wild type since the induction of expression of p53 in Cds1-/- ES cells was inhibited by caffeine similar to the wild type. Also, as a result of the cell cycle analysis by FACS, G2 arrest was observed in both Cds1(Chk2)+/- and Cds1(Chk2)-/ cells as shown in Fig. 2.

### [Example 8] Establishment of mouse embryonic fibroblasts derived from a mouse with which Cds1 gene was modified

The mouse embryos of post coitus day 12.5 to 14.5 were taken, and their heads and internal organs were excised. The embryos were cut with scissors and placed in tubes. Two milliliters of trypsin/EDTA was added to each tube. After an overnight incubation at 4°C, 2ml of DMEM containing 10% fetal bovine serum was added to each tube and stirred thoroughly with a pipette. As a result, a single cell suspension was prepared. Cells were recovered by centrifugation and cultured in DMEM medium containing 10% fetal bovine serum, 1 mM pyruvate sodium, 2 mM glutamine, 50 microM 2-mercaptoethanol, 0.1 mM of a non-essential amino acid, penicillin, streptomycin, and 4500 mg/L glucose.

### [Example 9] Analysis using thymocytes

After thymuses were taken out from Chk2-/- and p53-/- mice, they were dispersed in a medium to prepare thymocytes. After the thymocytes were double-stained with FITC-CD4 antibody and Rhodamine CD8 antibody, the distribution was analyzed by using a cell sorter. Chk2-/- and p53-/- thymocytes 24 and 48 hours after the irradiation of 10 Gy X ray were measured by double staining of annexin V and propidium iodide . Then, the survival rate of each of the cells compared to that of untreated cells was measured (Fig. 5).

### [Example 10] Analysis of the cell cycle using a cell sorter

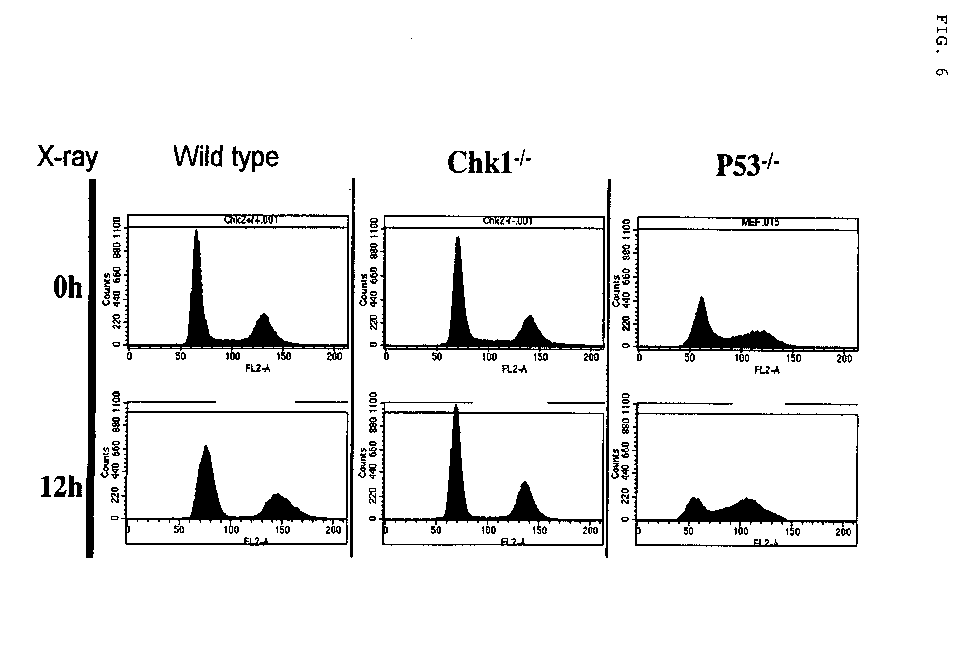

Embryonic fibroblasts of Chk2-/- and p53-/- mice were prepared, and the cells immediately before and 12 hours after the irradiation with 10Gy X ray were stained with propidium iodide using CycleTEST plus (cat No. 340242, Becton Dickinson). Then, the distribution of the cell cycle was analyzed using a cell sorter (Fig. 6).

### [Example 11] Evaluation of the survival rate of Cds1 (Chk2) -/- mouse against irradiation

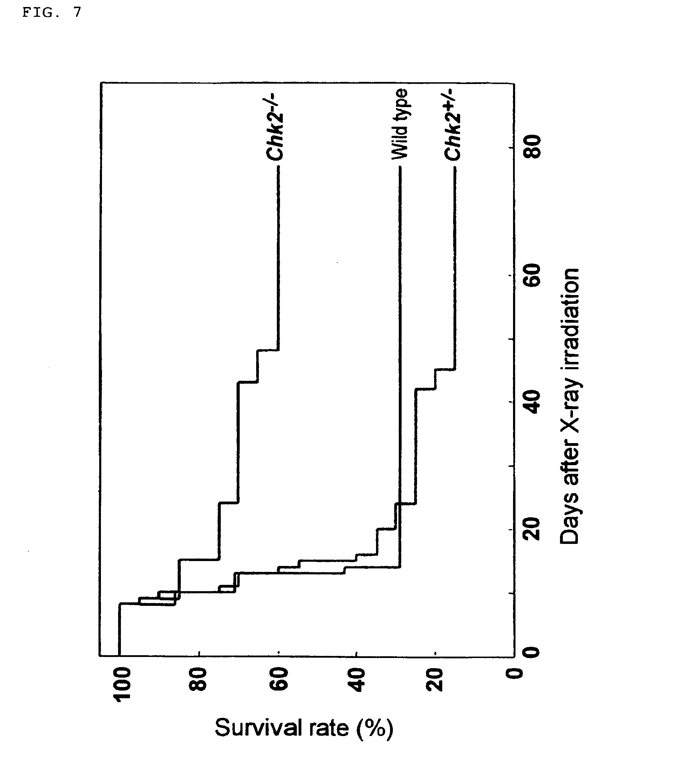

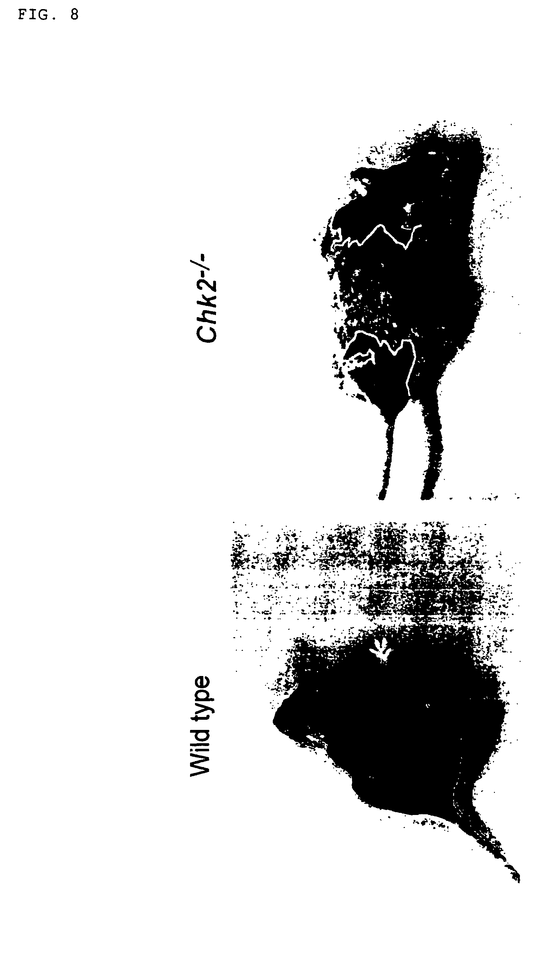

Cds1 (Chk2)-/-, Cds1 (Chk2)+/-, and wild type mice were irradiated and their survival rate was evaluated. As a result, Cds1 (Chk2)-/ mice had a lower death rate against the irradiation than Cds1 (Chk2) +/and wild-type mice and showed resistance against damage caused by irradiation (Fig. 7). Depigmentation of hair coats was observed in mice that escaped from death after the irradiation (Fig. 8).

### [Example 12] Histological test of Cds1 (Chk2)-/- mouse against irradiation

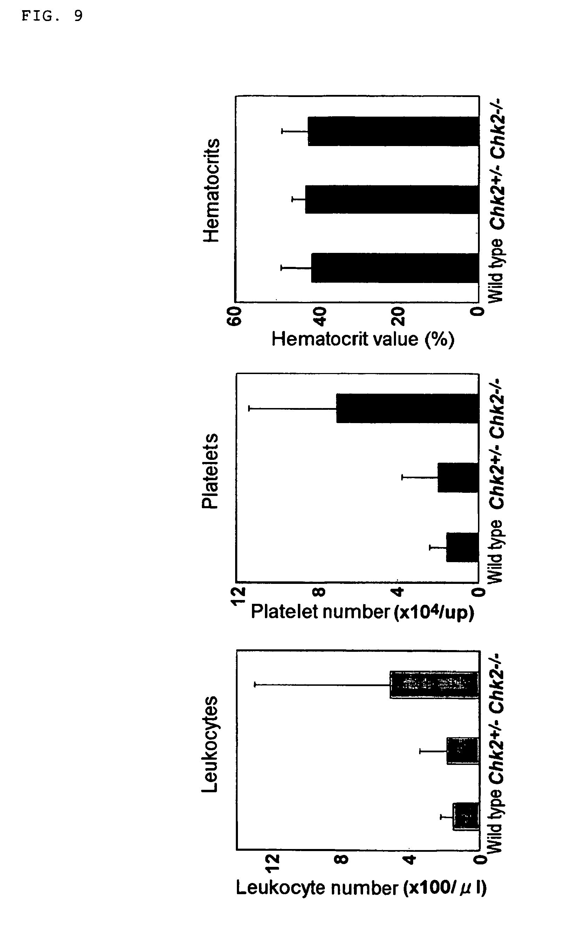

Cds1 (Chk2)-/-, Cds1 (Chk2)+/-, and wild type mice were irradiated, and a histological test was conducted. The change of the hematocrit value caused by the irradiation was not observed in any of the genotypes. However, an apparent decrease in the number of leukocytes and platelets was observed in all genotypes (Fig. 9).

### [Example 13] Histological test of Cds1 (Chk2)-/- mouse against irradiation

Cds1 (Chk2)-/-, Cds1 (Chk2) +/-, and wild type mice were irradiated, and histological tests of the spleen and bone marrow were conducted. The decrease in the number of hematopoietic cells was observed in the bone marrow of all genotypes. In the spleen of Cds1 (Chk2) +/- and wild-type mice, lymphocytes had disappeared and atrophy of the follicles was observed. However, atrophy of the follicles was not observed in the spleen of Cds1 (Chk2)-/- mice.

### [Example 14] Analysis of apoptosis induced by irradiation

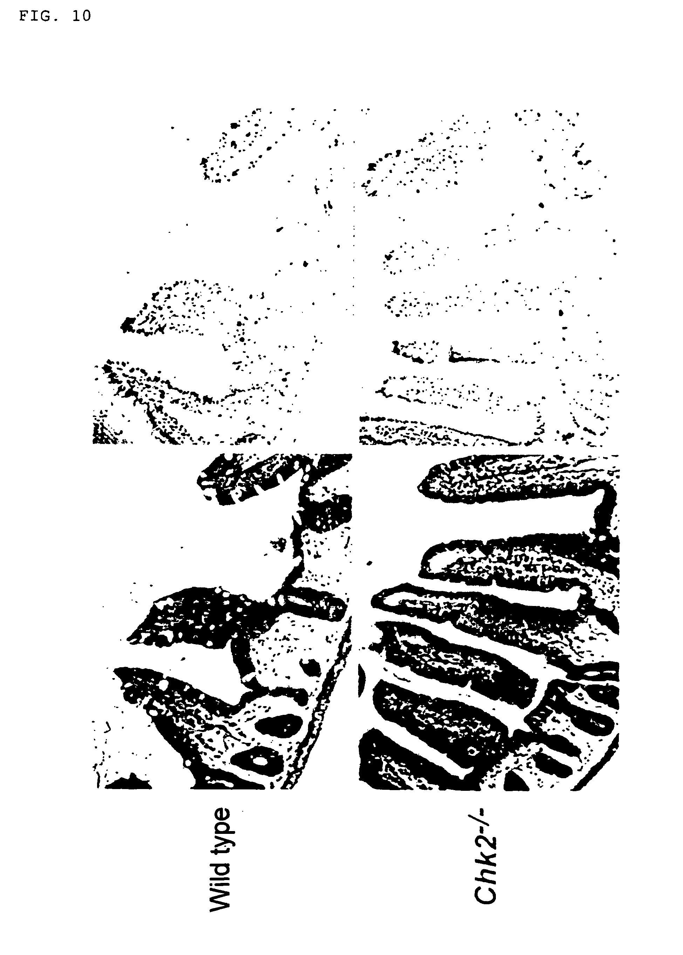

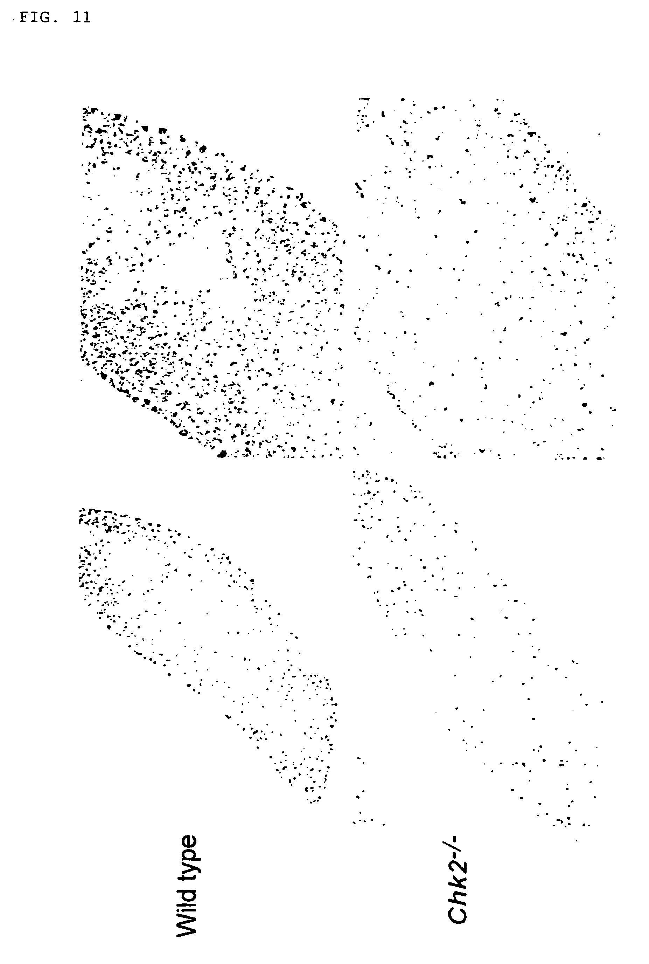

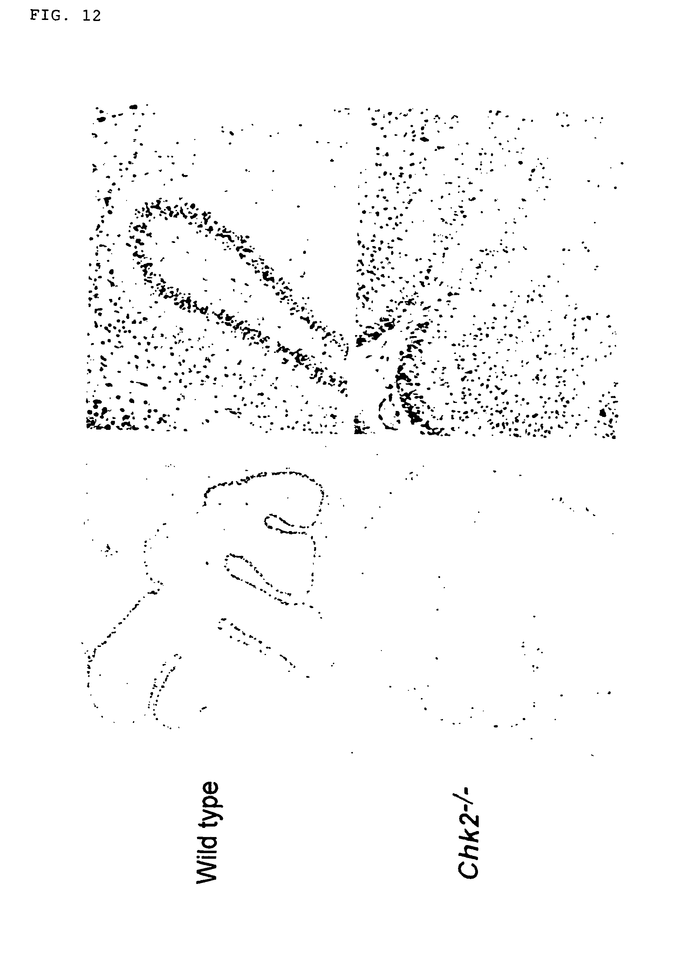

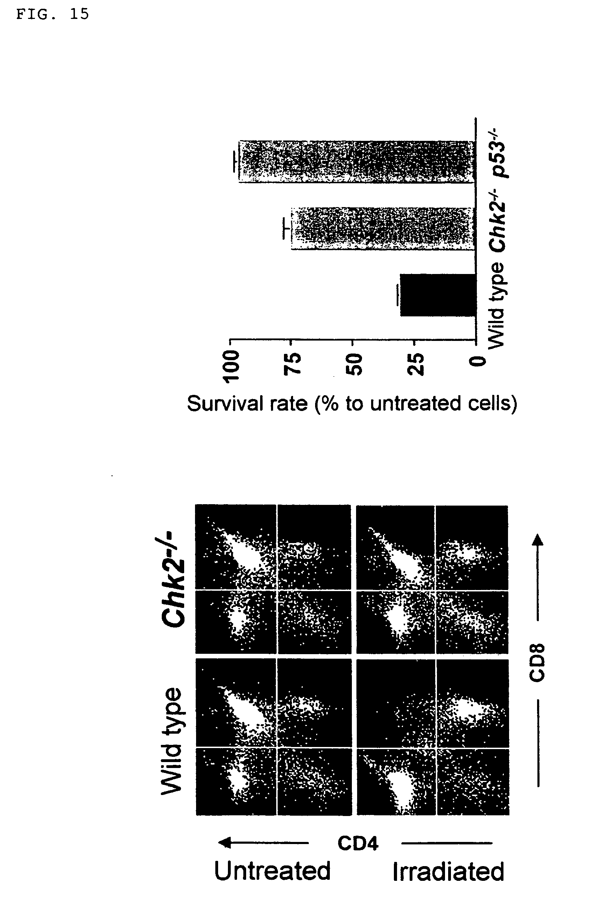

Cds1 (Chk2)-/- and wild-type newborn mice were irradiated, and apoptosis in the small intestine, thymus, and granule cell layer in the cerebellum was analyzed. A decrease in the number of cells showing apoptosis was observed in these tissues of Cds1 (Chk2)-/- mice (Fig. 10, 11, and 12). Also, Cds1 (Chk2)-/- and wild-type mice were irradiated, and their thymocytes were double-stained with antibodies against CD4 and CD8. Then, the distribution was analyzed by FACS. As a result, CD4/CD8 double-positive cells in wild-type mice showed sensitivity to irradiation, while those in Cds1-/- mice showed a decrease in sensitivity to irradiation similar to those in p53-/mouse (Fig. 15).

### [Example 15] Evaluation of the stabilization of p53 in thymocytes

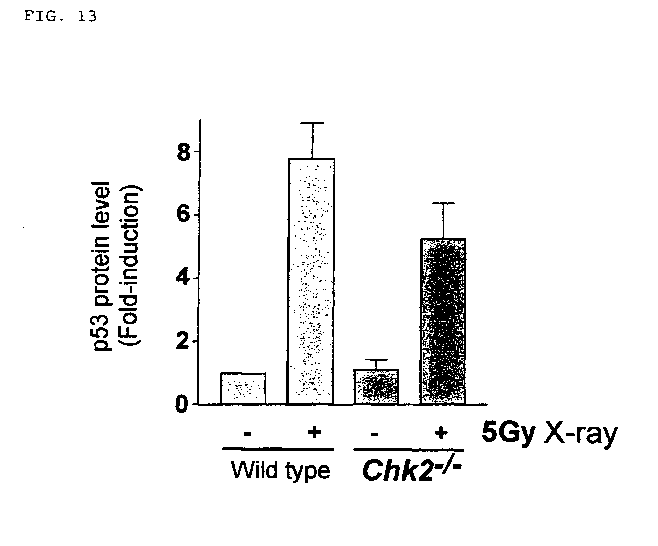

The expression of p53 was induced to thymocytes of Cds1(Chk2) -/- and wild-type mice by irradiation, and the expressed amount of p53 protein was investigated. As a result, it was shown that Cds1 (Chk2) -/- mice had few amounts of p53 protein compared with the wild-type mice (Fig. 13).

### [Example 16] Evaluation of gene expression in thymocytes

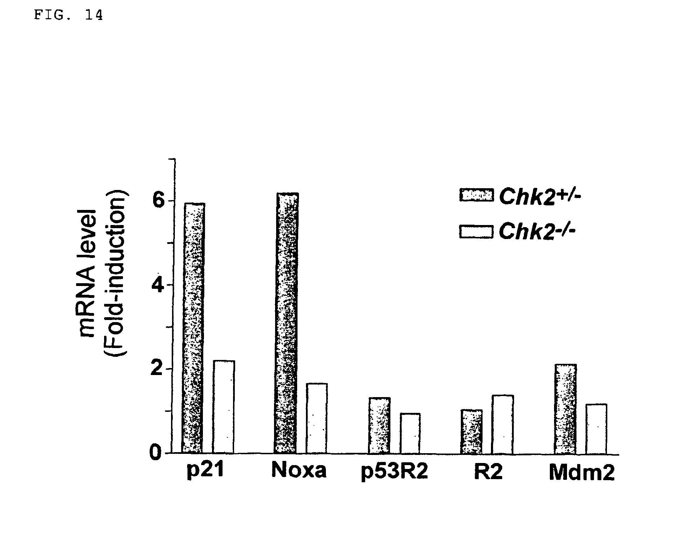

The expression of p53 was induced to thymocytes of Cds1(Chk2) -/- and wild-type mice by irradiation, and an expression of genes that are considered to be regulated by p53 was examined. As a result, a decrease in the expressions of p21 and Noxa was observed in Cds1 (Chk2)-/- mouse (Fig. 14).

### [Example 17] Observation of oncogenesis in Chk2-/- mice with and without irradiation

Chk2-/-, Chk2+/-, and Chk2+/+ mice were kept for long term, and it was observed whether tumors were formed or not. Mammary tumors were observed in six-month Chk2-/- mice, however, tumors were not observed in six-month Chk2+/- or Chk2+/+ mice.

Lymphomas were observed in all Chk2-/-, Chk2+/-, and Chk2+/+ mice with irradiation of 8Gy, at six months for Chk2+/- and Chk2+/+ mice, and at five months Chk2-/- mice.

### [Example 18] Evaluation of the survival rate of Chk2-/- · p53-/- mice against irradiation

Chk2+/- · p53+/- mice were obtained by mating or *in vitro* fertilization of Chk2+/- mice with p53-/- mice (Taconic). And then, Chk2-/- · p53-/- and Chk2+/+ · p53-/- mice were obtained by mating or *in vitro* fertilization of Chk2+/- · p53+/- mice with each other. These mice were irradiated with 4Gy, and their survival rate was investigated. As a result, the survival rate of Chk2+/+ · p53-/- mice was 6%, while that of Chk2-/- · p53-/- mice was 20% 28 weeks after the irradiation. These results showed that the deletion of the Chk2 gene can repress death of Chk2+/+ · p53-/- mice after irradiation.

### [Example 19] Histological test of Chk2-/- · p53-/- mouse against irradiation

Chk2-/- · p53-/-, Chk2+/+ · p53-/-, Chk2-/- · p53+/+, and Chk2+/+ · p53+/+ mice were irradiated with 8Gy, and histological test was conducted. An irradiation-induced change of the hematocrit value was not observed in any of the genotypes. However, an apparent decrease in the number of leukocytes and platelets was observed in all genotypes.

### [Example 20] Histological test of spleens of Chk2-/- · p53-/- mice against irradiation

Chk2-/- · p53-/-, Chk2+/+ · p53-/-, and Chk2+/+ · p53+/+ mice were irradiated with 8Gy, and a histological test of the spleens was conducted. Apparent atrophy in the B-cell zone and T-cell zone was observed in Chk2+/+ · p53+/+mice, however, little atrophy was observed in Chk2-/-· p53-/- and Chk2+/+· p53-/-mice. Hardly any germinal center was observed in the Chk2+/+ · p53+/+ and Chk2-/- · p53-/- mice, while a slightly obscure germinal center was observed in the Chk2+/+ · p53-/mice. The suppression of atrophy was dependent on the deletion of the p53 gene. There was no effect of deleting the Chk2 gene. Also, it was revealed that damages to the germinal center were suppressed depending on the deletion of the p53 gene, while the deletion of Chk2 gene canceled the suppression.

### [Example 21] Histological test of the thymus of Chk2-/- · p53-/- mice against irradiation

Chk2-/- · p53-/-, Chk2+/+ · p53-/-, and Chk2+/+ ·p53+/+ mice were irradiated with 8Gy, and histological test of the thymus was conducted. atrophy of thymus cortex was not observed in Chk2+/+ · p53+/+ mice, however, apparent atrophy was observed in Chk2-/- · p53-/- and Chk2+/+ · p53-/- mice. The atrophy was dependent on the deletion of the p53 gene. No effects of the deletion of Chk2 gene were observed. Atrophy of thymus medulla was observed in Chk2+/+ · p53+/+ and Chk2-/- · p53-/mice, however, suppression of atrophy was observed in Chk2+/+ · p53-/mice. It was revealed that atrophy of thymus medulla was suppressed depending on the deletion of p53 gene, while the deletion of Chk2 gene canceled the suppression.

### [Example 22] Histological test of bone marrow of Chk2-/- · p53-/- mice against irradiation

Chk2-/- · p53-/-, Chk2+/+ · p53-/-, and Chk2+/+ · p53+/+ mice were irradiated with 8Gy, and a histological test of the bone marrow was conducted. Atrophy of the bone marrow caused by irradiation was observed in all genotypes.

### Industrial Applicability

The present invention provides genetically modified mice in which both Cds1 (Chk2) alleles are inactivated and cell lines established from the genetically modified mice. The present invention further describes effects and side effects of anticancer drugs targeting DNA can be detected, and mechanisms of cell cycle arrest, apoptosis, and DNA repair that can be efficiently analyzed. Furthermore, the present invention describes the efficient screening of cell cycle-specific drugs.

## Claims

1. A genetically modified mouse, in which an exogenous gene is inserted to both of the Cds1 (Chk2) alleles.

2. The genetically modified mouse of claim 1, wherein the mouse has no Cds1 (Chk2) enzymatic activity.

3. A cell line established from the genetically modified mouse of claim 1 or 2.

## Patentansprüche

1. Genetisch veränderte Maus, in der ein exogenes Gen in beide Cds1 (Chk2)-Allele inseriert ist.

2. Genetisch veränderte Maus nach Anspruch 1, wobei die Maus keine Cds1 (Chk2)-Enzymaktivität hat.

3. Zelllinie, abgeleitet von der genetisch veränderten Maus nach Anspruch 1 oder 2.

## Revendications

1. Souris génétiquement modifiée, dans laquelle un gène exogène est inséré dans les deux allèles Cds1 (Chk2).

2. La souris selon la revendication 1, ladite souris n'ayant pas d'activité enzymatique de Cds1 (Chk2).

3. Lignée cellulaire établie à partir de la souris génétiquement modifiée selon la revendication 1 ou 2.
